Europäisches Patentamt

European Patent Office

Office européen des brevets

(11) Publication number: **0 346 622**
**A2**

# EUROPEAN PATENT APPLICATION

(21) Application number: 89108737.1

(22) Date of filing: 16.05.89

(51) Int. Cl.⁴: **A61K 9/00 , A61K 9/70 , A61K 39/35**

(30) Priority: 18.05.88 IT 2061888

(43) Date of publication of application:
20.12.89 Bulletin 89/51

(84) Designated Contracting States:
AT BE CH DE ES FR GB GR LI LU NL SE

(71) Applicant: NEO ABELLO' SpA
Via Falzarego 8
I-20021 Ospiate di Bollate (Milano)(IT)

(72) Inventor: Parmiani, Silvano, Dr.
Via Crescenzago, 24
I-20134 Milano(IT)

(74) Representative: Dragotti, Gianfranco et al
SAIC BREVETTI s.a.s. Viale Bianca Maria, 15
I-20122 Milano(IT)

(54) Pharmaceutical form and support for the administration of allergen extracts.

(57) For the administration of allergen extracts a pharmaceutical form for sublingual use is disclosed consisting of a shaped support, having an active surface which has to come into direct contact only with the sublingual mucosa, whereas the other surfaces thereof are sealed or waterproofed, said support being impregnated with a dosed amount of allergen extract.

EP 0 346 622 A2

## Pharmaceutical form and support for the administration of allergen extracts

The present invention relates to the sub-lingual administration of allergen extracts and particularly to a pharmaceutical form and to a device for such an administration. It is known that the therapy of the allergic diseases , besides with proper drugs (cortisone and derivatives thereof, anti-hystamine agents, compounds blocking the mastocitary degranulation, etc.) is carried out with allergen extracts , namely with extracts prepared starting from the same substance (pollen, animal dandruff, acary, etc.) which is responsible of the allergy.

The inhalatory route, the nasal topical route, the oral route, have been subsequently used after the parenteral administration, used at the beginning (Noon and Freeman, 1911).

Each of these routes has been proposed and used according to a number of possible variations.

(a) Parenteral route.

Administration, by subcutaneous or intradermal way, of allergens in form of:

a-1) aqueous solution
a-2) suspension in aluminium hydroxide
a-3) suspension in calcium sulphate
a-4) suspension in tyrosine
a-5) derivatives with formic aldehyde
a-6) derivatives with poliethylene glycol
a-7) derivatives with alginate
a-8) derivatives with glutaric aldehyde injected according to different posology schemes:
- scalar doses
- cluster doses brought closer
- rush doses
both as pre-season treatment and as perennial treatment.

b) Nasal topical route

Administration in form of rough dispersion aerosol of allergen in:

b-1) aqueous solution
b-2) fine powder.

In both cases the absorption takes place at the level of the nasal mucosa.

c) Inhalatory route

Administration , in form of micronized aerosol, of allergens in:

c-1) aqueous solution
c-2) fine powder

d) Oral route

Administration of allergens by oral route with preparations in form of:

d-1) aqueous solution
d-2) aqueousglycerol solution
d-3) enterosoluble tablets
d-4) gastroresistant capsules

For the preparations in the forms d-1 and d-2 the gastric and sub-lingual absorption routes have been used; for the preparations in the forms d-3 and d-4 the enteral absorption routes have been used.

The actually most practiced therapy is that by parenteral route with subcutaneous injections.

In any case the hyposensibiliring therapy, whatever of the said routes and forms is used, shows drawbacks, problems, side effects which, even if are acceptable and justified in comparison with the global benefit afforded by the therapy for the patient, represent for the patient and for the specialist doctor who prescribes and carries out the therapy a deterrent towards the use and the diffusion of the hyposensibilizing therapy.

It is useful to remember that the allergy is a syndrome having genetic base and which, as such, can not be removed by any drug, in the sense that no drug may modify the genetic constitution of the allergic human being; the possible therapies, consequently, are only aimed at prevent or reduce the allergic symptomatology without changing the cause by which it is originated.

It involves , in the case of use of drugs only, repeated administrations during each day and for the whole period during which the suffering situation of the patient exists.

Taking it into account that, in some cases, which by the way represent at least 50% of the total cases, the symptomatolgy is extended from 6 up to 12 months each year, it is evident that the therapy with drugs only is not practiceable.

The hyposensibilizing therapy, even if in turn is not able to modify the basic genetic defect, may induce at least in 80% of the treated cases a modification of the immunitary response of the organism, the benefits of which, although showing a decay in the long run , extend for more than the duration of the therapy and are active on all the symptoms (rhynitis, conjunctivitis, asthma ) in the case of the parenteral and oral route, on the rhynitis or on the asthma for the nasal topical route or the inhalatory route, respectively.

Whereas the drugs require one or more daily administrations, the immunotherapy by parenteral route is carried out by initial weekly injections and then with monthly injections.

The superiority of the immunotherapy with respect to the drugs, obviously, when the duration and seriousness of the symptoms are clearly relevant, is evident, this superiority being however attenuated by the above mentioned problems raised by to the immunotherapy..

It is also evident that, if these problems might be wholly or partially solved , the use of the immunotherapy would be much more widespread.

By detailedly analizing the problems related to each administration route for each form , the following problems, relating either to the route or to the form , can be identified.

Parenteral route.

The administration by injection route, which is necessarily carried out by the doctor, is per se traumatic: this fact alone rapresents already a first objectionable aspect, particularly in the case of children.

It is moreover evident that, in case side effects exist, it is impossible to avoid the diffusion of the injected hyposensibilizing solution, still present in the injection site, at the time at which the side effects become manifest (as caused by the already diffused portion).

The possible intervention to limit the side effects, intervention which require the immediate availability of a skilled doctor, must be practiced immediately and consist in acts by which the further diffusion is slowed or prevented (application of a haemostatic string to the leg in which the injection has been carried out, in a proximal position; injection, around the point at which the vaccine has been administered, of a vasoconstricting agent ) and in actions tending to pharmacologically nullify the same effect (administration, depending on the case, of anti-hystaminic agents, cortisone and derivatives thereof, by local and/or general route).

The serious general side effects are luckily rare and in most of the cases are caused by mistakes of the person carring out the injection, but the possibility of their occurrence represents a powerful psichological deterrent both for the doctor and for the patient.

None of the forms from a-1 to a-8 is exempt from the problem of the side effect even if for each form the incidence is different, it being maximum for the form a-1 and minimum for the forms a-5, a-6, a-7, a-8.

The form a-2, which anyhow is presently the most widespread one shows moreover the negative feature of leading, in some cases, to the forming of granulomes induced by aluminium hydroxide , which per se have no medical importance , but are haestetically disagreable.

Nasal topical route.

The administration of the allergen by nasal topical route causes the occurrence of the typical symptoms of the allergy at the nasal level.

Owing to the fact that the administration is carried out daily, it corresponds to a permanency of the symptomatology for the whole duration of the therapy.

This unavoidable circumstance, which is depending on the treatment itself, may be solved only by having recourse to preventive medications with substances preventing the appearance of local side effects.

Normally preventing medications with disodium chromoglycate, applied 30 minutes before the administration of the allergen extracts , are used.

Obviously, since the possible side effects of the nasal topical therapy are limited to the application area and are never of general type, the intrinsic risks of this therapy are practically absent.

The complexity of the proceedings, the occurrence of side effects , the daily frequence of the administrations, the efficacy limited to rhynites only, the need of anyhow administrating also a drug, render very low both the applicability of this type of therapy and the compliance to the same by the patient.

Inhalatory route.

The administration of the allergen by inhalatory route, so that the absorption takes place at the level of the bronchial mucosa, involves compulsorily the use of protecting drugs, such as disodium chromoglycate. In the absence of the administration of the drug, 30 minutes before the treatment with allergen, the patient would be likely to incur a very probable bronchial obstruction of a seriousness such as to even lead to the death.

In this case too if, despite the protection, complications would occur, it would not be possible to stop the absorption of the allergen already inhaled, with a further fast worsening of the symptoms.

The use of drugs may only be aimed at , it being obtained only in the best case, the disappearance of the bronchial obstruction whereby it must be anyhow very timely and carried out with very high competency and efficiency.

Consequently the administration must take place under strict medical control in specialized centers, account being also taken of the fact that the administration can be carried out only by means of proper equipments which are certainly not within the reach as all doctors and consequently available only in specialistic places.

Oral route.

The administration generically defined as oral route, as a matter of fact can be further classified as a function of the area of the digestive apparatus in which the allergen absorption takes place, the introduction thereof in the organism taking always place through the mouth.

It is thus possible to distinguish the following cases:

i) absorption at the gastric level.

The allergen is administered as an aqueous or water-glycerin solution, diluted with little water and taken by the fasted patient once a day according to a scalar dose scheme.

The absorption takes place at the gastric level, but consistent and well grounded doubts exist on the reliability and reproducibility of the absorption and on the efficiency thereof; as a matter of fact since the allergens are proteic molecules , in the stomac they might be denatured by the highly acidic pH which is found in this environnment and/or might be degraded by the proteolitic enzymes present, whereby in both cases. their activity would be either partially or totally.

Favourable results have been experienced only in the children and limitedely to some allergens; the latter fact can be explained by considering the losts of activity of the allergen molecules as a consequence of the pH or of the proteolitic enzymes which are found at the gastric levee and it can be more or less great as a function of a structure of the allergen proteins, which is different from one allergen to another.

Since the dose of administered allergen is absorbed at gastric level , in the case of side effects it is not possible to remove the not absorbed allergen, to avoid a strenghtening of the same side effects.

In this case too , as in the other ones, the intervention must be limited to the administration of drugs by which the same side effects are minimum.

The side effects are however rare and of modest entity; this fact permits the recourse to the therapy without the presence of the doctor.

(ii) Absorbtion at enteral level.

The allergen is administered in solid form either as soluble entero tablets or as gastroresistant capsules.

This form permits to avoid the problems connected to the environnment pH , since the enteral juice has a pH close to the neutrality, but not those connected to the enzymatic degradation, since also the enteral juice contains proteolitic enzymes.

The preparations useful for the enteral absorption contains very high dosages, greater by 1-2 orders of magnitude with respect to those used for the classic parenteral therapy, with very relevant problems of economical flessibility of production and consequently of marketing this kind of preparation.

The enteral absorption moreover is not devoid of side effects; the high dosage is furthermore a factor by which the same effects are increased.

Again, once the preparate has been ingested, it is impossible to stop the absorption taking place and consequently avoid a further worsening of the side effects when they occur.

All of that involves the necessity or advisability of carring out the therapy under medical control.

(iii) Absorption at sublingual level.

The allergen is administered as aqueous or water-glycerin solution , by putting the dose , in form of drops, in the sublingual area.

The posological scheme is of the type with increasing doses with daily administration and maintenance therapy, upon the maximum dose is achieved, which is carried out from daily trough three times a week.

The administration of the drugs by sublingual route is an already known method used in the medicine for some drugs; it is considered a quick and efficacious absorption route.

From the purely theoretical point of view, the sublingual route, owing to these characteristics, should involve relevant and frequent side effects when used for the administration of allergens; for them however there are normally used preparations and techniques permitting a slow absorption, (retard extracts, subcutaneous administration, etc.).

This fact explain the reluctance of the experimenters to follow this theoretically wrong route or anyhow a priori less favourable than other ones.

The clinical tests, carried out with all cautions, have instead shown the lack of ground of the fears, the optimum tolerability, the good therapeutical efficacy, the low frequency of side effects and anyhow their negligeable importance from the point of view of the seriousness, when the administration is carried out in correct manner and not absorbed portion of the extract is removed.

The sublingual vaccine is of relatively simple administration and, owing to the modest incidence of side effects and their negligeable importance and seriousness when the operation is carried out correctly, it might be administered without the assistency of a doctor, the necessity of the medical prescription and of periodical controls by a doctor being always compulsory.

The appearance of side effects moreover can be counteracted counter immediately and without difficulty , by removing the non absorbed part and stopping consequently the further absorption and the worsening of the effect.

Surprisingly , the local reactivity of the allergenic extract in the sublingual area is very reduced; other areas of the mucosa , and particularly the upper part of the tongue, the lips and the pharyngeal area are on the contrary relevantly reactive and in these areas local side effects occur, such as smart, reddening, itch , dryness sensation, swelling, if the administration is not strictly sublingual.

If the patient, by mistake or accidentally, does swallow the non absorbed part , risks of generalized side effects, such as vomiting, diarrhoea , asthmatic crysis, nettle-rash, exist.

Summing up, the situation reported in the following table 1, can be drawn:

| Administration route | Efficacy | Administration difficulty | Interval between two administration (days) | SIDE EFFECTS | | Necessity of administration and/or control of the doctor | Necessity of protecting medication |
|---|---|---|---|---|---|---|---|
| | | | | Frequency | Seriousness | | |
| Parenteral | Good | Simple | 7/30 | Average | Average | Yes | Not |
| Nasal Topical | Good | Simple | 0/1 | High in the absence of medication | Low | Not | Yes |
| Inhalatory | Doubtful | Complex | 0/1 | High in the absence of medication | Very high in the absence of medication | Yes (environment with specialistic equipments) | Yes |
| Oral — Gastric | Doubtful | Simple | 0/7 | Low | Low | Not | Not |
| Oral — enteral | Good only for some allergens | Simple | 0/7 | High | Average | Yes | Not |
| Oral — sub-lingual | Good | Simple | 0/2 | High | Low | Not | Not |

It is evident from the analysis of the several situations, that the sublingual therapy might be the preferable therapy, since a good efficacy is accompanied by the administration simplicity, the possibility of stopping at will the absorption of the administered dose in simple way by removing the allergen solution from the mouth of the patient, the lack of necessity of the doctor presence at the time of the administration (which is the condition for the self administration by the patient) , the low seriousness of the side effects.

The frequency of the side effects of the sublingual therapy, indicated as high, depends from a practical difficulty in carrying out in a stricktly correct manner the administration avoiding both the contact of the allergen solution with areas of the oral mucosa different from the sublingual one and the ingestion, by mistake or accidentally.

All these problems are found as the consequence of the fact that the administration takes place under liquid form and that with a relative easiness the liquid allergen may come into contact with areas different from the foreseen one.

The main purpose of the present invention is that of eliminating the problems and drawbacks as above mentioned and provide a pharmaceutical form which permits the sublingual administration of allergen extracts in a manner such that:

a) the contact between allergen extract and mucosa takes place exclusively in the sublingual area;

b) in whatever moment it is possible to stop this contact without permanency of residues and without diffusion of the allergen extracts towards other areas of the oral cavity.

Another purpose of the present invention is that of achieving the aforementioned purpose in a form which is simple from the point of view of the use and in a form which is consistent with the industrial production. These purposes are achieved by means of a pharmaceutical form for the sublingual administration of allergen extracts, characterized by comprising a support adapted to absorb the allergen extract in liquid form on an active or release surface, said support being shaped with a manoeuvering handle and a shovel part shaped correspondingly to the sublingual mucosa, with a recess matching with the tongue fraenum, said shovel part having the said active of releases surface of a material capable of absorbing a predetermined dose of allergen extract, the opposite surface of said shovel part being sealed or waterproofed so as to prevent any release of allergen extract to undesired areas of the oral cavity.

According to the preferred embodiment of the present invention said support or at least its shovel part is manufactured from an open cell foamed material, for example poliurethane, the non active surfaces thereof, which therefore are not useful for the release of allergen extracts towards the oral mucosa, are made waterproof by means of a plastic film, for example poliethylene, all these materials being selected among those known for biomedical or foodstuff use.

Alternatively to the foamed materials, the use of cellulosic vegetal fibers is foreseen, which are anchored onto a support of plastic material for biomedical or foodstuff use, for example poliethylene, and in that case the subject fibers cover only the surface of contact with the sublingual mucosa.

For the manufacturing of the pharmaceutical form according to the invention it is possible and preferred the embodiment according to which at the time of production of the pharmaceutical form the allergen extract is applied to the absorbing active surface, and thereafter the absorbed allergen extract is liophylized and then the active surface is protected with a seal tight protection fulfilling the sterility requisites but removable at the time of use.

Alternatively the support can be provided in the aforesaid manner apart from the absorption and from the liophilization of the allergen extract which is on the contrary applied in liquid form and with a suitable dosage to the said active surface at the time of use.

An embodiment of the support according to the invention is shown, for merely examplifying purpose, in the enclosed drawings in which:

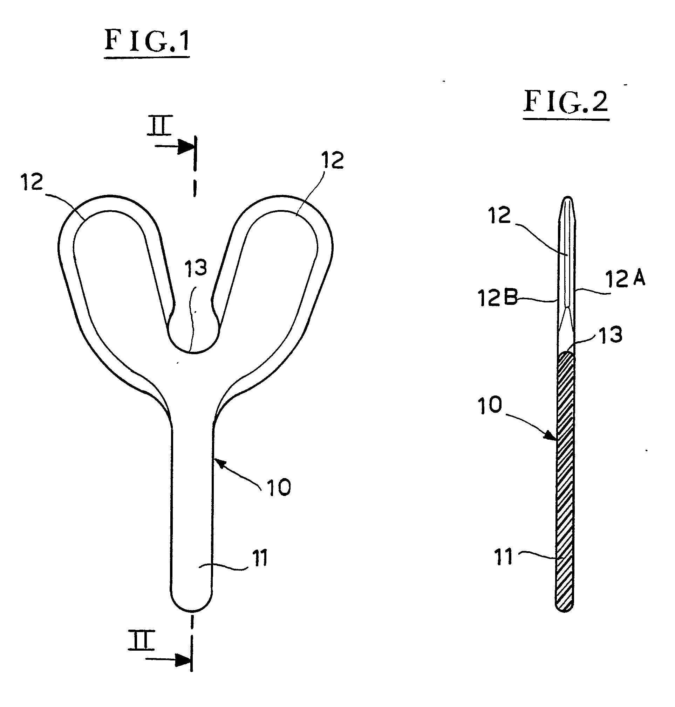

fig. 1 is a plan view of the support; and

fig. 2 is a cross-section view according to the line II-II of figure 1.

Referring to the drawings, the support 10 comprises a handle or stick 11 and a shovel part 12, shaped so as to have a recess 13, adapted to receive the tongue fraenum, when the active surface 12A of the shovel 12 is abutted under and against the sublingual mucosa.

As already mentioned the active surface 12A is of foamed material, for example poliurethane of foodstuff quality, whereas the opposite surface 12B is waterproof or waterproofed, for example by means of a poliethylene film it being to of foodstuff quality.

In the preferred embodiment the surface 12A is pre-impregnated with allergen extract in dosed amount, liophylized within the open cells of the foamed material.

At the time of use, once the packaging protecting material has been removed, the contact with the moisture of the sublingual mucosa permits the release of the allergen extract in dosed amount to the mucosa itself.

It is evident that for whatever occurrence the support 10 can be instantaneously extracted from the oral cavity, stopping totally the release of allergen extract.

It is possible to prepare supports with different amounts of allergen, so as to be able to administer increasing doses of allergen; each dose can be indicated by a different colour or numbering of the support.


EXAMPLE


A support is used of foamed poliurethane of foodstuff type , with open cells, closed in the lower part by a continuous films of foodstuff quality poliethylene , having a thickness of 1 mm, shaped as V with rounded edges, with a surface area of between about 1 and 1.5 cm.

The support is impregnated, at the absorbing side, with 0,5 ml of allergen solution containing, for example in the case of the pellitory respectively 40, 80, 160 , 320, 640 1,280 ng/ml of major allergen (Pj 10) of pellitory.

The supported liquid is liophylized according to well known techniques, an then the support is packaged so as to prevent the liophilizate from reabsorbing moisture.. The packing in blisters of thermoweeded envelopes of multilayer polyethylene/aluminium, has been found suitable.

Stability tests carried out on this preparation, according to the method known as RAST inhibition have demonstrated that 12 months after the preparation and packaging the preparation maintains at least 90% of the initial allergen activity.

Tests carried out on voluntary patients have permitted to assess that the release of at least 90% of the allergen molecules takes place within the first 5 minutes from the sublingual placing, without side effects.


Claims


1. Pharmaceutical form for the sublingual administration of allergen extract, characterized by comprising a support adapted to absorb the allergen extract in liquid form on an active or release surface, said support being shaped with a maneouvering handle and a shovel part which is shaped correspondingly to the sublingual mucosa with a recess for the adaption to the tongue fraenum, said shovel part having said active or release surface of a material capable of absorbing a predetermined dose of allergen extract, the opposite surface of said shovel part being seal tight or waterproof, so as to prevent whatever release of allergen extract to undesired areas of the oral cavity.

2. Pharmaceutical form according to claim 1, characterized in that the material capable of absorbing allergen extract is selected among open cell foamed plastic material, of biomedical or foodstuff type, and fibers of cellulosic material.

3.Pharmaceutical form according to claim 2, characterized in that said foamed plastic material is open cell foamed polyurethane.

4. Pharmaceutical form according to claim 1, characterized in that said seal tightness or waterproofing consists of a plastic film of biomedical or foodstuff type.

5. Pharmaceutical form according to claim 4, characterized in that said film is of foodstuff poliethylene.

6. Pharmaceutical form according to claim 1, characterized in that said allergen extract is absorbed on said material capable of absorbing it and liophylized in situ, it being then protected by a protection removable at the time of use.

7. Pharmaceutical form according to claim 1, characterized in that said allergen extract is in liquid form which is absorbed in said material capable of absorbing it at the time of use.

8. Pharmaceutical form according to claim 1, characterized in that said support has a handle or stick and a shovel part having a recess housing the tongue fraenum.

FIG.1

FIG.2